# FASCICULE DE BREVET EUROPEEN

(11) **EP 2 126 059 B1**
(45) Date de publication et mention de la délivrance du brevet: **12.09.2012**
(21) Numéro de dépôt: 08762131.4
(22) Date de dépôt: 20.02.2008
(51) Int. Cl.: C12N 9/04, C12N 9/02, C12R 1/545, C12P 7/22, C12P 7/24, C12P 7/42

(54) **SYSTEME DE PRODUCTION DE MOLECULES AROMATIQUES CHEZ STREPTOMYCES**
VERFAHREN ZUR HERSTELLUNG AROMATISCHER MOLEKÜLE IN STREPTOMYCETEN
METHOD FOR PRODUCING AROMATIC MOLECULES IN STREPTOMYCES

(30) Priorité: 21.02.2007 FR 0701229
(43) Date de publication de la demande: 02.12.2009
(73) Titulaire: V. Mane Fils, 06620 Bar sur Loup (FR)
(72) Inventeur: LAMBERT, Fanny, F-06580 Pegomas (FR); ZUCCA, Joseph, F-06130 Grasse (FR); MANE, Jean, F-06130 Grasse (FR)
(74) Mandataire: Cabinet Plasseraud
(86) Numéro de dépôt international: PCT/FR2008/050284
(87) Numéro de publication internationale: WO 2008/113936

(56) Documents cités:
- EP-A1- 0 885 968
- WO-A-2007/099230
- WO-A-2007/099231
- WO-A2-01/55342
- OVERHAGE ET AL: "Harnessing eugenol as a substrate for production of aromatic compounds with recombinant strains of Amycolatopsis sp. HR167" JOURNAL OF BIOTECHNOLOGY, ELSEVIER SCIENCE PUBLISHERS, AMSTERDAM, NL, vol. 125, no. 3, 18 septembre 2006 (2006-09-18), pages 369-376, XP005605038 ISSN: 0168-1656 cité dans la demande
- OVERHAGE J ET AL: "Highly efficient biotransformation of eugenol to ferulic acid and further conversion to vanillin in recombinant strains of Escherichia coli" APPLIED AND ENVIRONMENTAL MICROBIOLOGY, WASHINGTON,DC, US, vol. 69, no. 11, novembre 2003 (2003-11), pages 6569-6576, XP002410833 ISSN: 0099-2240 cité dans la demande
- RAINER PLAGGENBORG ET AL: "Potential of Rhodococcus strains for biotechnological vanillin production from ferulic acid and eugenol" APPLIED MICROBIOLOGY AND BIOTECHNOLOGY, SPRINGER-VERLAG, BE, vol. 72, no. 4, 19 janvier 2006 (2006-01-19), pages 745-755, XP019441618 ISSN: 1432-0614
- BENEN J A E ET AL: "Molecular cloning, sequencing, and heterologous expression of the vaoA gene from Penicillium simplicissimum CBS 170.90 encoding vanillyl-alcohol oxidase" JOURNAL OF BIOLOGICAL CHEMISTRY, AMERICAN SOCIETY OF BIOLOCHEMICAL BIOLOGISTS, BIRMINGHAM,, US, vol. 273, no. 14, 3 avril 1998 (1998-04-03), pages 7865-7872, XP002410834 ISSN: 0021-9258
- FRAAIJE M W ET AL: "SUBSTRATE SPECIFICITY OF FLAVIN-DEPENDENT VANILLYL-ALCOHOL OXIDASE FROM PENICILLIUM SIMPLICISSIMUM EVIDENCE FOR THE PRODUCTION OF 4-HYDROXYCINNAMYL ALCOHOLS FROM 4-ALLYLPHENOLS" EUROPEAN JOURNAL OF BIOCHEMISTRY, BERLIN, DE, vol. 234, 1995, pages 271-277, XP000986081 ISSN: 0014-2956

## Description

La présente invention concerne la production de molécules aromatiques naturelles. Plus particulièrement, la présente invention concerne la bioconversion dans une bactérie de substrats, de préférence l'eugénol, en dérivés phénoliques notamment l'alcool coniférylique et l'acide férulique, lesdits dérivés étant utilisables pour la production de vanilline naturelle employée en aromatique alimentaire ou en parfumerie (arômes ou fragrances).

La vanilline (3-methoxy-4-hydroxybenzaldehyde) est le composant principal responsable des propriétés olfactives et gustatives de l'extrait de vanille dérivé de gousses de *Vanillia planifolia.* Il s'agit de l'une des molécules aromatiques les plus utilisées dans l'industrie. Cependant, la production de vanilline naturelle à partir de gousse de vanille ou d'extrait de vanille ne couvre que 20% de ce marché ; son utilisation est limitée en raison d'une part du potentiel de gousses disponibles au niveau mondial et d'autre part en raison du prix élevé, très fluctuant, de ces gousses (de l'ordre de 30 €/kg à 450 €/kg soit au minimum 1500 €/kg de potentiel en vanilline naturelle).

La vanilline de synthèse est donc fréquemment utilisée comme un substituant bon marché (environ 15 €/kg) de la vanilline naturelle pour des applications en parfumerie, en cosmétique et dans les industries agro-alimentaires. Les arômes de synthèse sont toutefois moins appréciés par les consommateurs que les arômes naturels.

C'est pourquoi l'on cherche à obtenir des molécules aromatiques naturelles, en particulier la vanilline, par des procédés biologiques, notamment de bioconversion, qui mettent en oeuvre des microorganismes tels que les bactéries.

Au sens de la présente invention, on entend par bioconversion la transformation biologique d'un substrat, de préférence issu d'une source naturelle, pour obtenir des arômes, des fragrances ou des précurseurs d'arômes ou de fragrances naturels.

La vanilline peut être produite suivant le schéma réactionnel suivant :

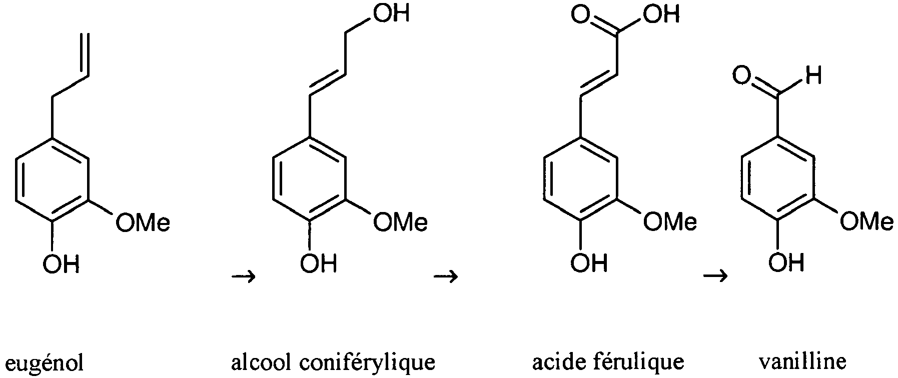

Plusieurs procédés de production de molécules naturelles telle que la vanilline ont déjà été décrits dans l'art antérieur.

Notamment, le brevet EP 0885968 décrit un procédé de production de vanilline chez une bactérie appartenant au genre Streptomyces. Ce procédé comprend: a) la culture dans un milieu approprié d'une bactérie appartenant au genre Streptomyces de façon à former un bouillon de fermentation, b) l'addition dans ledit bouillon de 5 à 40 gL-1 d'acide férulique, de manière à produire de la vanilline, et c) l'extraction de la vanilline et du gaïacol produits.

Un autre exemple décrit dans l'article J. Overhage et al. divulgue 1) l'expression du gène de la vanillyl alcool oxydase dans Escherichia Coli et 2) l'expression des gènes calA (codant pour la coniféryl alcool déshydrogénase) et calB (codant pour la coniféryl aldéhyde déshydrogénase). Ce procédé permettrait la production d'acide férulique comme produit principal de la conversion d'eugénol par la souche recombinante finale E. Coli XL1 Blue (pSKvaom-PcalAmcalB) (Highly efficient biotransformation of eugenol to ferulic acid and further conversion to vanillin in recombinant strains of *Escherichia coli,* 2003, Applied and Environmental microbiology p 6569-6576). Il est indiqué que l'acide férulique produit par cette souche pourrait servir de substrat à une seconde souche multirecombinée E. Coli (pSKechE/Hfcs) pour produire de la vanilline naturelle (J. Overhage et al. Appl. Env. Microbiol. 65: 4837-4847, 1999)

WO 2007/099230 décrit un procédé de production d'acide férulique, d'alcool coniférulique et/ou de vanilline naturels à partir d'eugénol dans la levure mais ne fait aucunement mention du genre *Streptomycetaceae* comme hôte potentiel pour l'expression hétérologue du gène vao.

WO 01/55342 décrit différentes séquences d'acides nucléiques de synthèse, optimisées en vue d'accroître leur expression en système hétérologue. Plus particulièrement, ce document divulgue la séquence synthétique du gène vaoA (SEQ ID N°67 page 30, lignes 33-38) qui est par ailleurs identique à 84% à la séquence optimisée SEQ N°1 et à 78% à la séquence optimisée SEQ ID N°8 prise dans toute sa longueur. Toutefois, dans ce document, les gènes synthétiques sont obtenus en vue d'une expression augmentée chez les bactéries entériques (dont E Coli) mais pas chez *Streptomycetaceae.*

Un dernier exemple de production de dérivés phénoliques à partir d'eugénol a été décrit dans l'article « Harnessing eugenol as a substrate for production of aromatic compounds with recombinant strains of Amycolatopsis sp HR167 » (J Biotechnol. 2006 Sep 18;125(3):369-76. Epub 2006 May 4. Overhage J, Steinbuchel A, Priefert H. ). Cet article divulgue l'expression du gène de la vanillyl alcool oxydase dans la souche Amycolatopsis sp HR167 permettant de catalyser la conversion d'eugénol en alcool coniférylique, aldéhyde coniférylique, acide férulique, gaïacol, acide vanilliqueCes trois exemples de production de précurseurs de vanilline naturelle ou de vanilline naturelle elle-même présentent l'inconvénient pour le premier d'avoir un coût de production relativement élevé du fait de l'utilisation d'acide férulique purifié qui est une matière première onéreuse, et pour les autres d'être difficiles à mettre en oeuvre industriellement du fait 1) de l'utilisation de souches multirecombinées non alimentaires, 2) du faible rendement des bioconversions.

Pour qu'un procédé biologique employant des microorganismes puisse être rentable, il est préférable d'utiliser un substrat très disponible et très bon marché et un microorganisme inoffensif et stable génétiquement. Les inventeurs ont ainsi cherché à mettre au point un procédé de production de précurseurs de vanilline naturelle ou de vanilline naturelle elle-même, qui soit simple à mettre en oeuvre industriellement, c'est-à-dire ne comportant de préférence qu'une seule étape, et dont le coût de production soit moindre que celui de l'art antérieur. La solution proposée par l'invention est d'utiliser un substrat naturel disponible et de faible coût qui est l'eugénol ainsi qu'une souche de classe 1 appartenant au genre Streptomyces, telle que par exemple Streptomyces griseus.

Ainsi, la présente invention a pour objet un procédé de fabrication de vanilline ou de précurseurs de vanilline tels que l'acide férulique ou l'alcool coniférylique, ou encore d'un mélange comprenant les précurseurs de la vanilline et de la vanilline, par bioconversion d'eugénol, dans une bactérie appartenant au genre Streptomyces tel que défini à la revendication 1. Dans un premier mode de réalisation, le procédé de l'invention permet de fabriquer de la vanilline. Dans un second mode de réalisation, le procédé de l'invention permet de fabriquer de l'acide férulique. Dans un troisième mode de réalisation, le procédé de l'invention permet de fabriquer de l'alcool coniférylique. Dans un quatrième mode de réalisation, le procédé de l'invention permet de fabriquer un mélange d'alcool coniférylique, d'acide férulique et/ou de vanilline.

Le procédé de l'invention est mis en oeuvre à l'aide d'une bactérie appartenant au genre Streptomyces comprenant la séquence SEQ ID NO :1 ou la séquence SEQ ID NO :8 ou toute séquence nucléotidique ayant au moins 70%, de préférence 80%, très préférentiellement 90% d'identité avec la séquence SEQ ID NO : 1 ou toute séquence ayant au moins 80%, de préférence 90% d'identité avec la séquence SEQ ID NO :8. Les séquences SEQ ID NO :1 et SEQ ID NO :8 correspondent au gène de la vanillyl alcool oxydase présent dans le génome du champignon filamenteux Penicillium simplicissimum, ledit gène ayant été optimisé pour être lu dans une bactérie appartenant au genre Streptomyces.

La présente invention a donc pour avantage de permettre la production en une seule étape, via un catalyseur biologique, de vanilline naturelle, d'alcool coniférylique et/ou d'acide férulique naturels, essentiellement dépourvu d'impureté et de bas prix.

Un autre objet de l'invention est une bactérie appartenant au genre Streptomyces comprenant au moins une séquence nucléotidique SEQ ID NO :1 ou SEQ ID NO 8 ou toute séquence nucléotidique ayant au moins 70%, de préférence 80%, très préférentiellement 90% d'identité avec la séquence SEQ ID NO :1 ou toute séquence ayant au moins 80%, de préférence 90% d'identité avec SEQ ID NO :8.
De préférence, la bactérie est Streptomyces griseus.

Un autre objet de l'invention est la séquence nucléotidique SEQ ID NO :1 et la séquence SEQ ID NO :8. La séquence originelle de la vanillyl alcool oxydase est présente dans le génome du champignon filamenteux Penicillium simplicissimum. Les codons présents dans l'ADN de Penicillium simplicissimum ne peuvent pas être utilisés pour la transcription par une bactérie appartenant au genre Streptomyces. Les inventeurs ont donc remplacé ces codons par des codons appropriés lus par une bactérie appartenant au genre Streptomyces, de façon à obtenir une séquence nucléotidique optimisée (SEQ ID NO :1 et SEQ ID NO :8).

Un autre objet de l'invention est un vecteur d'expression comprenant au moins une séquence nucléotidique SEQ ID NO :1 ou au moins une séquence SEQ ID NO :8.
On entend par « vecteur » ou « vecteur d'expression » toute séquence d'ADN dans laquelle il est possible d'insérer des fragments d'acide nucléique étranger, les vecteurs permettant d'introduire de l'ADN étranger dans une cellule hôte. Des exemples de vecteurs sont les plasmides, les cosmides, les chromosomes artificiels de levures (YAC), les chromosomes artificiels de bactéries (BAC) et les chromosomes artificiels dérivés du bactériophage P1 (PAC) ou les vecteurs dérivés de virus.

Dans un mode de réalisation de l'invention, le vecteur d'expression comprenant la séquence nucléotidique SEQ ID NO :1 ou la séquence SEQ ID NO :8 est un vecteur bifonctionnel qui est spécifique des bactéries appartenant au genre Streptomyces.

On entend par vecteur bifonctionnel tout plasmide capable de se répliquer dans deux organismes hôtes différents car il porte deux origines de réplication différentes et peut, par conséquent, être utilisé pour transférer des gènes d'un hôte à un autre. Au sens de la présente invention, un vecteur bifonctionnel spécifique des bactéries appartenant au genre Streptomyces est un vecteur comprenant des moyens permettant son expression dans lesdites bactéries. De préférence, le vecteur bifonctionnel comprend un gène oriT qui permet une conjugaison inter générique et notamment une expression aussi bien dans E. Coli que dans une bactérie appartenant au genre Streptomyces.

Très préférentiellement, le vecteur d'expression utilisé dans l'invention, comprend: 1) des séquences (soit un gène d'intégration soit un gène de réplication) permettant l'introduction et le maintien de la séquence SEQ ID NO :1 ou de la séquence SEQ ID NO :8 dans la souche, 2) un gène de résistance à un antibiotique, 3) des séquences permettant l'excision du marqueur de sélection dans la souche industrielle finale, 4) une origine de transfert oriT de type RP4 permettant la conjugaison intergénique entre E. Coli et Streptomyces, 5) un promoteur et 6) un terminateur forts pour l'expression de la séquence SEQ ID NO :1 ou SEQ ID NO :8 et 7) une séquence MCS.

Dans un mode de réalisation de l'invention, le vecteur d'expression est choisi parmi un vecteur intégratif ou un vecteur hautement réplicatif de type multicopie. De préférence, le vecteur est choisi parmi les vecteurs intégratifs pFLA2 et pFLA3. Ces vecteurs intégratifs ont l'avantage de permettre une intégration simple ou multiple d'un gène d'intérêt dans la souche finale. L'excision du marqueur de sélection permet de réutiliser le même vecteur pour transformer à nouveau la souche et augmenter ainsi le nombre de copie du gène d'intérêt.

Selon une autre préférence, le vecteur hautement réplicatif de type multicopie est le vecteur pFLA4. Ce vecteur présente l'avantage de se répliquer de nombreuses fois dans l'hôte, assurant ainsi un grand nombre de copie du gène d'intérêt dans l'hôte.

Un autre objet de l'invention est un procédé de production d'acide férulique, d'alcool coniférylique et/ou de vanilline, comprenant la bioconversion d'eugénol par une bactérie appartenant au genre Streptomyces comprenant au moins une séquence nucléotidique SEQ ID NO :1 ou SEQ ID NO :8 ou toute séquence nucléotidique ayant au moins 70%, de préférence 80%, très préférentiellement 90% d'identité avec la séquence SEQ ID NO :1 ou toute séquence ayant au moins 80%, de préférence 90% d'identité avec SEQ ID NO :8.

Ladite bactérie est obtenue par :
a) clonage de la séquence nucléotidique SEQ ID NO :1 ou SEQ ID NO :8 correspondant au gène de la vanillyl alcool oxydase dans un vecteur d'expression, de préférence spécifique des bactéries appartenant au genre Streptomyces,
b) transformation du vecteur d'expression obtenu en a) dans une bactérie compétente, de préférence E. Coli DH5α,
c) transfert du vecteur d'expression comprenant la séquence SEQ ID NO :1 ou la séquence SEQ ID NO :8 (après tests d'activité sur l'enzyme produite) dans E. Coli ET12567 (pUZ8002)
d) transformation ou conjugaison d'une bactérie appartenant au genre Streptomyces avec la bactérie obtenue en c).
e) sélection des meilleurs transformants obtenus en d) en présence du substrat eugénol.

Selon un mode de réalisation de l'invention, la séquence SEQ ID NO :1 ou la séquence SEQ ID NO :8 est clonée dans un vecteur d'expression, ledit vecteur étant de préférence un vecteur bifonctionnel spécifique des bactéries appartenant au genre Streptomyces. Pour ce faire, la séquence nucléotidique SEQ NO :1 ou SEQ ID NO :8 comprise dans un vecteur de type pUC57 et ledit vecteur d'expression sont dans un premier temps digérés par une ou plusieurs enzymes de restriction. La séquence SEQ ID NO :1 ou SEQ ID NO :8 est ensuite insérée par simple ligation ou tout autre moyen d'insertion dans le vecteur d'expression.

Des bactéries du type E. Coli DH5α sont transformées selon n'importe quel procédé connu avec le produit de ligation (vecteur d'expression comprenant la séquence SEQ ID NO :1 ou SEQ ID NO :8). Les bactéries contenant le vecteur d'expression sont ensuite sélectionnées grâce au marqueur de sélection, de préférence un gène de résistance aux antibiotiques, présent sur le vecteur d'expression. Avantageusement, ce marqueur de sélection est le gène de résistance au thiostrepton.

Avantageusement, le vecteur d'expression est ensuite introduit par transformation dans des bactéries compétentes, de préférence la souche E. Coli ET12567. Les transformants sont ensuite sélectionnés selon la méthode décrite ci-dessus.

Enfin, le transfert du gène de la vanillyl alcool oxydase dans une bactérie appartenant au genre Streptomyces peut se faire par conjugaison ou transformation. Lesdites bactéries comprenant le gène de la vanillyl alcool oxydase intégré ou non dans leur génome sont sélectionnées selon la méthode décrite ci-dessus.

Les bactéries appartenant au genre Streptomyces et comprenant le gène de la vanillyl alcool oxydase sont alors cultivées dans les conditions permettant l'expression dudit gène et en présence d' eugénol.

De préférence, les bactéries sont incubées dans un milieu dont le pH est inférieur à 10, de préférence compris entre 6 et 9,5, et préférablement compris entre 7 et 9, et plus préférablement compris entre 7,5 et 8,5 ; et dont la température est comprise entre 27 et 40°C, de préférence entre 27 et 39°C, plus préférablement entre 29 et 37°C et encore plus préférablement entre 36 et 37°C, voire est égale à 37°C.

Dans un mode de réalisation préféré, le procédé de l'invention comprend :
1) la culture d'une bactérie appartenant au genre Streptomyces comprenant au moins une séquence nucléotidique SEQ ID NO :1 ou SEQ ID NO :8 ou toute séquence nucléotidique ayant au moins 70%, de préférence 80%, très préférentiellement 90% d'identité avec la séquence SEQ ID NO :1 ou toute séquence ayant au moins 80%, de préférence 90% d'identité avec SEQ ID NO :8,
2) l'addition en une seule dose d'eugénol comme substrat de bioconversion dans le milieu de fermentation dans une quantité comprise entre 5 à 40g/l, de préférence 10 à 35 g/l,
3) l'extraction de l'alcool coniférylique du milieu de fermentation.

Selon ce procédé visant à obtenir un taux de conversion majoritaire en alcool coniférylique, le substrat eugénol est de préférence ajouté en une seule dose dans le milieu de culture de ladite bactérie et la quantité d'eugénol introduite est comprise entre 5 et 40 g/l, de préférence du 10 à 25 g/l. De préférence, la solution d'eugénol ajoutée comprend un mélange d'eugénol, de glucose et d'huile ou un mélange d'eugénol, de glycérol et d'huile. De préférence, lesdites bactéries sont incubées dans un milieu dont le pH est compris entre 8 et 10, de préférence entre 7 et 9 et plus préférentiellement est égale à 9. L'aération du milieu est de préférence comprise entre 0.1 et 1 L d'air/min. L'agitation du milieu est de préférence inférieure à 1000 rpm pour 3 L de milieu, et très préférentiellement est de 900 rpm pour 3 L de milieu. La température du milieu est de préférence comprise entre 27 et 40°C, de préférence entre 27 et 39°C, plus préférentiellement entre 27 et 37°C et très préférentiellement est de 37°C.

Dans ces conditions, le taux de conversion de l'eugénol en alcool coniférylique est généralement compris entre environ 50 et 95% pour une quantité de substrat introduite variant de 10 à 25 g/l. L'alcool coniférylique est produit à hauteur de 10 à 25 g/l.

Dans un mode de réalisation préféré, le procédé de l'invention comprend :
1) la culture d'une bactérie appartenant au genre Streptomyces comprenant au moins une séquence nucléotidique SEQ ID NO :1 ou SEQ ID NO :8 ou toute séquence nucléotidique ayant au moins 70%, de préférence 80%, très préférentiellement 90% d'identité avec la séquence SEQ ID NO :1 ou toute séquence ayant au moins 80%, de préférence 90% d'identité avec SEQ ID NO :8,
2) l'addition en continu d'eugénol comme substrat de bioconversion dans le milieu de fermentation dans une quantité comprise entre 5 à 30g/l, de préférence 10 à 25 g/l,
3) l'extraction de l'acide férulique du milieu de fermentation.

Selon ce procédé visant à obtenir un taux de conversion majoritaire en acide férulique, le substrat est de préférence ajouté en continu dans le milieu de culture de ladite bactérie et la quantité d'eugénol finale introduite est comprise entre 5 et 30 g/l, de préférence du 10 à 25 g/l. De préférence, la solution d'eugénol ajoutée comprend un mélange d'eugénol, de glucose et d'huile ou un mélange d'eugénol, de glycérol et d'huile. De préférence, la solution d'eugénol est ajoutée en continu dans le milieu sur 3 à 5 jours avec un débit compris entre 0,2 g/L/H et 0,12 g/L/H.

De préférence, lesdites bactéries sont incubées dans un milieu dont le pH est compris entre 8 et 10, de préférence entre 7 et 9 et plus préférentiellement entre 7 et 8.5. L'aération du milieu est de préférence comprise entre 0.1 et 1 L d'air/min. L'agitation du milieu est de préférence inférieure à 1000 rpm pour 3 L de milieu, et très préférentiellement est de 900 rpm pour 3 L de milieu. La température du milieu est de préférence comprise entre 27 et 40°C, de préférence entre 27 et 39°C, plus préférentiellement entre 27 et 37°C et très préférentiellement est de 37°C. Le débit de substrat dans le fermenteur de 3L est compris entre 0.05 g d'eugénol/L/heure et 1 g d'eugénol/L/heure. Préférentiellement, le débit d'eugénol est fixé entre 0.15 et 0.7 g d'eugénol/L/ heure. Dans ces conditions, le taux de conversion en acide férulique est généralement compris entre environ 50 et 95% pour une quantité de substrat introduite variant de 10 à 25 g/l. L'acide férulique est produit à hauteur de 10 à 25 g/l.

Dans un mode de réalisation préféré, le procédé de l'invention comprend :
1) la culture d'une bactérie appartenant au genre Streptomyces comprenant au moins une séquence nucléotidique SEQ ID NO :1 ou SEQ ID NO :8 ou toute séquence nucléotidique ayant au moins 70%, de préférence 80%, très préférentiellement 90% d'identité avec la séquence SEQ ID NO :1 ou toute séquence ayant au moins 80%, de préférence 90% d'identité avec SEQ ID NO :8,
2) l'addition en continu ou en une seule dose d'eugénol comme substrat de bioconversion dans le milieu de fermentation dans une quantité comprise entre 5 à 50g/l, de préférence 10 à 35 g/l,
3) l'extraction de la vanilline du milieu de fermentation.

Selon ce procédé visant à obtenir un taux de conversion le plus élevé possible en vanilline naturelle, lesdites bactéries sont, de préférence, incubées dans un milieu dont le pH est inférieur à 10, de préférence compris entre 7 et 9, et plus préférentiellement entre 7,5 et 8,5. L'aération du milieu est de préférence comprise entre 0.1 et 1 L d'air/min. L'agitation du milieu est de préférence inférieure à 1000 rpm pour 3 L de milieu, et très préférentiellement est de 900 rpm pour 3 L de milieu. La température du milieu est de préférence comprise entre 27 et 40°C, de préférence entre 27 et 39°C, plus préférentiellement entre 27 et 37°C et très préférentiellement est de 37°C.

De préférence, la quantité d'eugénol ajoutée au milieu de culture de ladite bactérie est comprise entre 5 à 50 g/l, de préférence de 10 à 35 g/l. L'addition d'eugénol peut se faire en continu ou en une seule dose. De préférence, la solution d'eugénol ajoutée comprend un mélange d'eugénol, de glucose et d'huile ou un mélange d'eugénol, de glycérol et d'huile. De préférence, lorsque la solution d'eugénol est ajoutée en continu dans le milieu, l'ajout se fait sur 3 à 5 jours avec un débit compris entre 0,2 g/L/H et 0,12 g/L/H.

Le taux de conversion en vanilline est compris entre 30 et 72% pour une quantité d'eugénol distribué variant de 10 à 35 g/l.

Un autre objet de l'invention est un procédé de production de vanilline à partir d'acide férulique par la même bactérie appartenant au genre Streptomyces. En effet, la souche possède naturellement le patrimoine génétique et enzymatique lui permettant d'exécuter cette réaction. Ainsi, qu'elle contienne ou non la séquence SEQ ID NO :1 ou SEQ ID NO :8, elle est capable de convertir l'acide férulique en vanilline. On peut en effet vérifier que dans des conditions appropriées, la quantité de vanilline produite peut être comprise entre 10 et 25 g/l, de préférence entre 14 et 24 g/l lorsqu'on administre à la souche entre 28 et 36 g/l d'acide férulique.

Selon un autre mode de réalisation de l'invention, l'étape de conversion de l'acide férulique en vanilline est réalisée par voie enzymatique ou voie biochimique, notamment selon les procédés décrits dans les brevets EP 0 606 441 et EP 0 804 606.

### EXEMPLES

La présente invention sera mieux comprise à l'aide du complément de description qui va suivre, qui se réfère à des exemples d'obtention de vecteurs d'expression comprenant la séquence SEQ ID NO :1, de bactéries appartenant au genre Streptomyces comprenant ces vecteurs d'expression et de leur utilisation pour la production, par bioconversion de l'eugénol, d'acide férulique et de vanilline.

Dans les exemples qui suivent, donnés à titre illustratif, il sera fait référence aux figures en annexe, dans lesquelles :
la figure 1 correspond au vecteur d'expression pFLA2,
la figure 2 correspond au vecteur d'expression pFLA3,
la figure 3 correspond au vecteur d'expression pFLA4.

### 1- Obtention de la séquence SEQ ID NO :1 correspondant à la vanillyl alcool oxydase optimisée pour la transcription dans des bactéries appartenant au genre Streptomyces.

Certains codons du gène de la vanillyl alcool oxydase présent dans Penicillium simplicissimum sont probablement non lus par les bactéries appartenant au genre Streptomyces. On considère comme codons « probablement non lus » les codons qui ont une fréquence d'utilisation dans les bactéries appartenant au genre Streptomyces inférieure à 10 pour 1000. La fréquence d'utilisation des codons est décrite par la base de données « codon usage database » (http://www.kasuka.or.jp/codon/). D'après la fréquence inventeurs ont créé une séquence optimale SEQ ID NO :1 pour l'expression de la vanillyl alcool oxydase dans ces cellules.

Des sites de restriction ont été introduits en 5' et 3' pour permettre le clonage de la séquence dans un vecteur. Les sites de restriction ajoutés sont fonction des sites de restriction présents dans les zones de clonage multi sites (MCS) des vecteurs.

A l'extrémité 3' de la séquence sont également introduits les codons STOP et la queue poly-histidine permettant une purification rapide de l'enzyme produite sur colonne Ni-NTA.

La séquence SEQ ID NO :1 optimisée est synthétisée dans le plasmide pUC57 par la société GenScript.

### 2- Construction de quatre vecteurs d'expression comprenant la séquence SEQ ID NO :1 : pFLA2, pFLA3 et pFLA4.

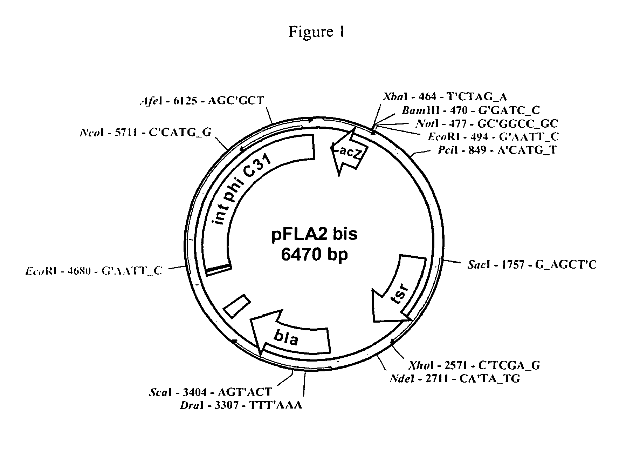

### Construction de pFLA2 (figure 1) :

Le plasmide pRES19 a été utilisé comme source du gène bla codant pour la beta-lactamase. Le gène bla a été amplifié par PCR avec les amorces bla1F (5'CTCGAGAGACGAACTCCTTGAACC3', SEQ ID NO :2) et bla1R (5'GGCCTTACCAATGCTTAATCAG3', SEQ ID NO :3) pour obtenir un fragment de 1,4 kb digéré par les enzymes de restriction XhoI et HaeIII. De la même manière, le gène tsr du plasmide pANT849 a été amplifié par les amorces tsr1F (5'**GAGCTC**TGACTGAGTTGGACAC3', SEQ ID NO :4) et tsrlR (5'**CTCGAC**TTATCGGTTGGCCG3', SEQ ID NO :5) et le fragment de 821 bp obtenu a été digéré par les enzymes SacI et XhoI. Les fragments d'ADN *bla* 1.4kb (aux extrémités XhoI / HaeIII) et *tsr* 819 bp (aux extrémités XhoI / NdeI) ont été clonés dans le vecteur pSET152 digéré par les enzymes de restriction NaeI et SacI pour former pFLA2b.

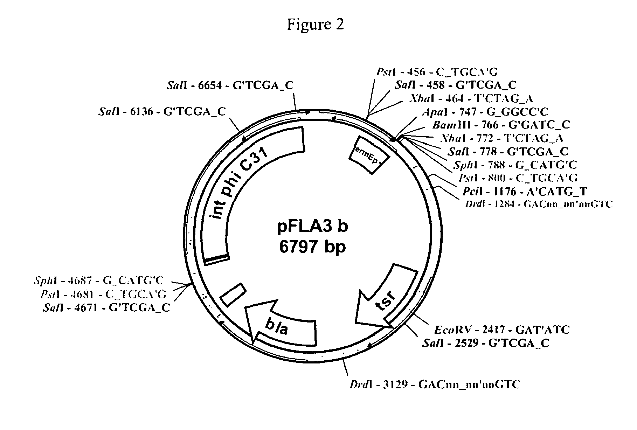

### Construction de pFLA3 (figure 2) :

Dans le vecteur d'expression pFLA3b, le gène LacZ de pFLA2b a été remplacé par un promoteur fort de Streptomyces : le promoteur ermEp*. Le gène ermEp* a été synthétisé dans le vecteur pUC57 par la société GenScript Corporation et l'insert a été isolé par digestion du vecteur (pU57-ermEp) avec les enzymes de restriction XbaI et EcoRI.

Le vecteur pFLA2b a été également digéré par les enzymes de restriction XbaI et EcoRI afin d'ouvrir la séquence LacZ et de cloner le promoteur ermEp* par simple ligation entre les deux sites de coupure.

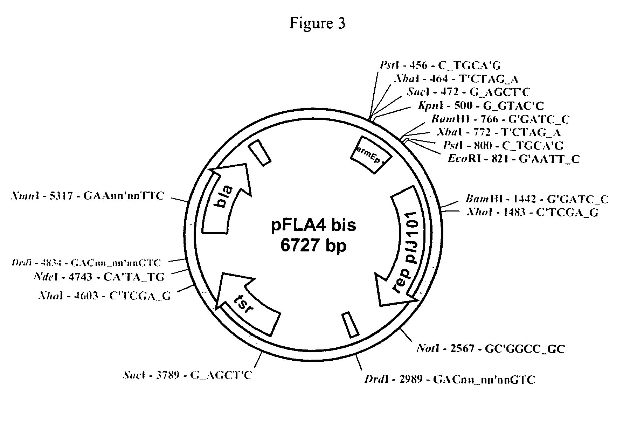

### Construction de pFLA4 (figure 3) :

Le vecteur pFLA4b est construit à partir du vecteur pFLA3b. Les gènes int phiC31 et attP sont excisés du vecteur pFLA3 par une digestion endonucléasique AfeI et SbfI.

L'extrémité cohésive (3') générée par SbfI est rendue franche par l'action de la nucléase S1 et les deux extrémités sont raccordées par simple ligation. 1771 bp sont ainsi éliminées du vecteur pFLA3b pour obtenir le vecteur pFLA3-2b. Un fragment nucléotidique de 1.8kb comprenant le gène rep, permettant au vecteur une réplication autonome dans la souche hôte, est amplifié par PCR sur le vecteur pIJ101 isolé de Streptomyces lividans en utilisant les amorces rep1F (5'ACATGTGTTAGTGCGAAGTGGGC3', SEQ ID NO :6) et resp1R (5'CTGCGAGTTCAG3', SEQ ID NO :7). Les produits de PCR ont été digérés par les endonucléases PciI et DrdI et l'insert ainsi obtenu a été cloné dans le vecteur pFLA3-2b entre les sites PciI et DrdI pour obtenir le vecteur pFLA4b de 6727 bp.

La séquence SEQ ID NO :1 comprend les extrémités BamHI (GGATCC) (5') et XbaI (3'), lorsque le clonage est prévu dans le vecteur pFLA2.

La séquence SEQ ID NO :1 comprend les extrémités ApaI (5') et BamHI (GGATCC) (3'), lorsque le clonage est prévu dans le vecteur pFLA3.

La séquence SEQ ID NO :1 comprend les extrémités KpnI (GGTACC) (5') et XbaI (TCT AGA) (3'), lorsque le clonage est prévu dans le vecteur pFLA4.

### 3- Transfert de la séquence SEQ ID NO :1 dans une bactérie appartenant au genre Streptomyces

Des bactéries E. Coli DH5α sont transformées avec un des vecteurs construits précédemment comprenant la séquence SEQ ID NO :1. Puis ces vecteurs sont dans un second temps introduits dans la souche E. Coli ET12567 (pUZ8002) rendue compétente. Cette souche est déficiente en méthylation ce qui évite les restrictions sur l'ADN lorsque celui-ci est introduit dans une bactérie appartenant au genre Streptomyces.

La sélection des transformants obtenus se fait en présence d'antibiotique Apramycine : les souches résistantes sont celles qui comprennent la séquence SEQ ID NO :1.

Le transfert de la séquence SEQ ID NO :1 dans Streptomyces griseus se fait ensuite par conjugaison. Les exconjugants sont de nouveau sélectionnés pour leur résistance à l'apramycine. Les souches Streptomyces 92873 et Streptomyces sp 92286 ont été transformées avec un des vecteurs construits précédemment et deux souches recombinantes ont été sélectionnées. Ces souches, comprenant au moins une copie de la séquence SEQ ID NO :1 sont nommées respectivement Streptomyces sp 92873 et Streptomyces sp 92286). Une estimation du niveau de production de la vanillyl alcool oxidase a été réalisée en effectuant des cultures de la souche transformée sur milieu Strepto et une analyse SDS-PAGE des extraits totaux de bactérie. L'analyse confirme la présence d'une protéine de la taille attendue de 64 KDa. La quantité produite est estimée entre 0.5 et 5 µg de VAO par ml de culture pour un poids sec de 8 g/L de cellules.

### 4- Activité enzymatique de la vanillyl alcool oxydase exprimée dans les souches d'E. Coli DH5α comprenant la séquence SEQ ID NO :1

Les cellules bactériennes sont cultivées à partir d'un stock conservé à -80°C et l'expression de la VAO est constitutive due aux promoteurs forts utilisés dans les vecteurs de clonage. Les cellules sont ensuite sonifiées et l'extrait total (Fraction T) centrifugé pour éliminer les débris cellulaires et montrer la présence de la VAO dans la fraction soluble. L'analyse SDS-PAGE montre que la protéine VAO dont la taille théorique attendue est d'environ 64.7kDa est bien produite. Elle est majoritairement soluble. La protéine clonée comportant une queue polyhistidinique à son extrémité N-Terminal, elle est purifiée sur colonne de nickel et le niveau d'expression est analysé en SDS-PAGE. La quantité de vanillyl alcool oxydase produite dans E. Coli varie de 2 à 5 µg par ml de culture pour une DO de 2,3 à 600 nm.

L'activité enzymatique de la vanillyl alcool oxydase est analysée par conversion de l'alcool vanillique et de l'eugénol avec la protéine purifiée sur colonne Ni NTA à partir de 10ml de culture de bactéries E. Coli comprenant la séquence SEQ ID NO :1. Les produits aromatiques dérivés de ces conversions sont analysées en HPLC.

On a pu déterminer que l'enzyme produite était bien la vanillyl alcool oxidase. Le taux de transformation avec l'enzyme purifiée est compris entre 85 et 92%. On mesure 0.74mM de vanilline formé à partir de 0.8mM d'alcool vanillique et 0.69mM d'alcool coniférylique formé à partir de 0.8mM d'eugénol.

L'alcool vanillique est converti en vanilline que l'enzyme soit purifiée ou que la réaction ait lieu sur un moût de cellules E .Coli DH5α. Le pH optimal pour la conversion est situé entre 7 et 10 et dans ces conditions, la quantité de vanilline produite varie de 0.41 à 1g/l à partir de 0.5 à 1g/l d'alcool vanillique distribué.

La vanillyl alcool oxydase catalyse aussi la conversion de l'eugénol en alcool coniférylique. Le gène cloné dans E.Coli DH5α codant pour la VAO transforme jusqu'à 10 g/l d'eugénol en alcool coniférylique avec un rendement de 90% dans un milieu à pH 10. La réaction est efficace aussi bien avec l'enzyme purifiée qu'avec un moût de cellules recombinantes E. coli DH5α.

### 5 - Sélection des souches recombinantes comprenant la séquence SEQ ID NO :1

La purification de l'enzyme par colonne Ni NTA et l'analyse sur gel SDS PAGE montrent que la protéine produite par les transformants des souches Streptomyces sp 92873 ou Streptomyces sp 92286 est bien la vanillyl alcool oxydase.

Les bactéries transformées avec le vecteur d'expression contenant la vanillyl alcool oxidase, telles que décrites précédemment, ont été sélectionnées pour leur capacité à consommer l'eugénol et à le convertir en vanilline, en acide férulique ou en alcool coniférylique suivant les conditions expérimentales. Les souches Streptomyces sp 92286 et Streptomyces sp 92873 ont été sélectionnées comme étant les meilleurs candidats. Les tests en erlemeyers avec des souches Streptomyces comprenant la séquence SEQ ID NO :1 montrent des taux de consommation de l'eugénol variant de 75 à 90% selon les conditions de bioconversion.

Les conditions de bioconversion en erlenmeyers sont les suivantes :

Les bactéries transformées sont cultivées sur milieu complet gélosé (YPD) puis repiquées dans 100 ml de milieu Strepto liquide jusqu'en fin de phase exponentielle de croissance dans les conditions suivantes : 35°C, 175 rpm. Le substrat est alors ajouté au milieu après reprise des cellules dans un tampon phosphate 0.5M, pH8.5 à hauteur de 2.5 g/l d'eugénol.

Les conditions de bioconversion sont les suivantes : 35°C, 175 rpm, 48H. Un suivi journalier permet de calculer les rendements de bioconversion. Les analyses sont menées par HPLC. Tous les dérivés phénoliques sont mesurés et quantifiés.

La quantité d'acide vanillique ou de vanilline synthétisée par le meilleur candidat est de l'ordre de 1 à 2 g/l à partir de 2.5 g/l d'eugénol fourni en début de bioconversion.

### 6 - Conversion d'eugénol en vanilline naturelle par une souche Streptomyces comprenant la séquence SEQ ID NO :1

La bioconversion d'eugénol est optimisée en fermenteur de 3 litres. Le taux de conversion en 4 jours est compris entre 30 et 72% pour une quantité de substrat distribué variant de 10 à 25 g/l. Les conditions optimales de pH pendant la conversion sont les suivantes : le pH est régulé entre 7 et 10 et préférentiellement entre 7 et 9.

L'aération est comprise entre 0.1 et 0.8 volume d'air /volume de milieu/ min. L'agitation est de 900 rpm pour 3L de milieu et la température régulée à 37°C pendant les phases de culture et de conversion.

Un exemple de conditions de fermentation est le suivant :

Jour 1 : Mise en culture de la souche transformée 92873 comprenant la séquence SEQ ID NO :1 dans un milieu Strepto (extrait de levure : 0.8%, glucose 3.2%,) à pH7.5 pendant 48 heures en fermenteur de 3L, 37°C, 900 rpm, avec une aération de 0.3vvm.

Jour 3 : Lorsque le taux d'oxygène dissout dans le milieu de culture est stabilisé à 100% depuis au moins 4 heures et que la mesure du taux de glucose dans le milieu indique que celui-ci est complètement épuisé, entre 0.04% et 0.08% de MgSO₄ sont ajoutés dans le milieu.

Addition du substrat :

L'addition de l'eugénol est faite en continu sur 3 jours (pour 15 g/l d'eugénol, addition de 15 g/l de glucose + 200 g d'huile) avec un débit 0.2 g/l/H ou sur 5 jours (le débit est alors de 0.12 g/l/H. Les conditions de fermentations sont les suivantes : pH régulé entre 7 et 9, aération comprise entre 0.2 à 1 L air / min.

Jour 6 ou 8 : Arrêt de la conversion quand les résultats chromatographiques confirment la fin de la biotransformation.

Un suivi journalier permet de calculer les rendements de bioconversion. Les analyses sont menées par HPLC. Tous les dérivés vanilliques sont mesurés et quantifiés.

Un autre exemple de conditions de fermentation est le suivant :

Jour 1 : Mise en culture de la souche transformée 92873 comprenant la séquence SEQ ID NO :1 dans un milieu Strepto (extrait de levure : 0.8%, glucose 3.2%,) à pH7.5 pendant 48 heures en fermenteur de 3L, 37°C, 900 rpm, avec une aération de 0.3vvm.

Jour 3 : Lorsque le taux d'oxygène dissout dans le milieu de culture est stabilisé à 100% depuis au moins 4 heures et que la mesure du taux de glucose dans le milieu indique que celui-ci est complètement épuisé, entre 0.04% et 0.08% de MgSO₄ sont ajoutés dans le milieu.

Addition du substrat :

L'addition de l'eugénol est faite en continu sur 5 jours (pour 20 g/l d'eugénol, addition de 10g/l de glucose + 85 g/l d'huile) avec un débit 0.17 g/l/H. Les conditions de fermentations sont les suivantes : pH régulé entre 7 et 9, aération comprise entre 0.2 à 1 L air / min.

Jour 6 ou 8 : Arrêt de la conversion quand les résultats chromatographiques confirment la fin de la biotransformation.

Un suivi journalier permet de calculer les rendements de bioconversion. Les analyses sont menées par HPLC. Tous les dérivés vanilliques sont mesurés et quantifiés.

Un autre exemple de conversion est le suivant :

Jour 1 : Mise en culture de la souche transformée 92873 comprenant la séquence SEQ ID NO :1 dans un milieu Strepto (extrait de levure : 0.8%, glucose 3.2%, MgSO₄ entre 0.04% et 0.08%) à pH7.5 pendant 48 heures en fermenteur de 3L, 37°C, 900 rpm, avec une aération de 1 L air/min.

Jour 3 : Lorsque le taux d'oxygène dissous dans le milieu de culture est stabilisé à 100% depuis au moins 4 heures et que la mesure du taux de glucose dans le milieu indique que celui-ci est complètement épuisé, entre 0.04% et 0.08% de MgSO₄ sont ajoutés dans le milieu.

Addition du substrat :

L'eugénol est ajouté en 1 seule dose (pour 10 g/l d'eugénol ajout de 10 g/l de glucose) avec les conditions de fermentation suivantes : le pH est régulé entre 7 et 9 avec de la soude, l'aération est comprise entre 0.1 à 0.33 vvm.

Jour 5 : Arrêt de la conversion quand les résultats chromatographiques confirment la fin de la biotransformation.

Un suivi journalier permet de calculer les rendements de bioconversion. Les analyses sont menées par HPLC. Tous les dérivés vanilliques sont mesurés et quantifiés.

### 7- Conversion d'eugénol en acide férulique naturel par une souche Streptomyces comprenant la séquence SEQ ID NO :1

La bioconversion d'eugénol en acide férulique est optimisée en fermenteur de 3 litres. Le taux de conversion en 5 jours est compris entre 30 et 90% pour une quantité de substrat distribué variant de 10 à 25 g/l. Les conditions optimales de pH pendant la conversion sont les suivantes : le pH est régulé entre 7 et 10 et préférentiellement à 7.

L'aération est comprise entre 0.1 et 0.8 volume d'air /volume de milieu/ min. L'agitation est de 900 rpm pour 3L de milieu et la température régulée à 37°C pendant les phases de culture et de conversion.

Un exemple de conditions de fermentation est le suivant :

Jour 1 : Mise en culture de la souche transformée 92873 comprenant la séquence SEQ ID NO :1 dans un milieu Strepto (extrait de levure : 0.8%, glucose 3.2%,) à pH7.5 pendant 48 heures en fermenteur de 3L, 37°C, 900 rpm, avec une aération de 0.3vvm.

Jour 3 : Lorsque le taux d'oxygène dissous dans le milieu de culture est stabilisé à 100% depuis au moins 4 heures et que la mesure du taux de glucose dans le milieu indique que celui-ci est complètement épuisé, entre 0.04% et 0.08% de MgSO₄ sont ajoutés dans le milieu.

Addition du substrat :

L'addition de l'eugénol est faite en continu sur 4 jours (pour 15 g/l d'eugénol, addition de 15 g/l de glucose + 200 g d'huile) avec un débit 0.15g/l/H. Les conditions de fermentations sont les suivantes : pH régulé à 7, aération comprise entre 0.2 à 1 L air / min.

Jour 7 : Arrêt de la conversion quand les résultats chromatographiques confirment la fin de la biotransformation.

Un suivi journalier permet de calculer les rendements de bioconversion. Les analyses sont menées par HPLC. Tous les dérivés vanilliques sont mesurés et quantifiés. On peut obtenir un mélange vanilline, alcool coniférylique, acide férulique.

### 8- Conversion d'eugénol en alcool coniferylique naturel par une souche Streptomyces comprenant la séquence SEQ ID NO :1

La bioconversion d'eugénol est optimisée en fermenteur de 3 litres. Le taux de conversion en 2 jours est compris entre 40 et 95% pour une quantité de substrat distribué variant de 10 à 25 g/l. Les conditions optimales de pH pendant la conversion sont les suivantes : le pH est régulé entre 7 et 10 et préférentiellement entre 7 et 9.

L'aération est comprise entre 0.1 et 0.8 volume d'air /volume de milieu/ min. L'agitation est de 900 rpm pour 3L de milieu et la température régulée à 37°C pendant les phases de culture et de conversion.

Un exemple de conversion est le suivant :

Jour 1 : Mise en culture de la souche transformée 92873 comprenant la séquence SEQ ID NO :1 dans un milieu Strepto (extrait de levure : 0.8%, glucose 3.2%, MgSO₄ entre 0.04% et 0.08%) à pH7.5 pendant 48 heures en fermenteur de 3L, 37°C, 900 rpm, avec une aération de 1 L air/min.

Jour 3 : Lorsque le taux d'oxygène dissous dans le milieu de culture est stabilisé à 100% depuis au moins 4 heures et que la mesure du taux de glucose dans le milieu indique que celui-ci est complètement épuisé, entre 0.04% et 0.08% de MgSO₄ sont ajoutés dans le milieu.

Addition du substrat :

L'eugénol est ajouté en 1 seule dose (pour 10 g/l d'eugénol ajout de 10 g/l de glucose) avec les conditions de fermentation suivantes : le pH est régulé entre 8 et 9 avec de la soude 5M, l'aération est comprise entre 0.1 à 0.33 vvm.

Jour 4 ou 5 : Arrêt de la conversion quand les résultats chromatographiques confirment la fin de la biotransformation.

Un suivi journalier permet de calculer les rendements de bioconversion. Les analyses sont menées par HPLC. Tous les dérivés vanilliques sont mesurés et quantifiés.

### 9- Exemple d'extraction de la vanilline du moût de bioconversion.

En fin de culture, le moût est concentré sous vide partiel puis extrait au Methyl Cyclohexane à chaud. Après concentration du solvant, la vanilline cristallise. Elle est lavée à l'éthanol et recristallisée de façon à obtenir une pureté HPLC supérieure à 99,5%.

## Revendications

1. Procédé de production d'acide férulique, d'alcool coniférylique et/ou de vanilline naturels, comprenant la bioconversion d'eugénol par une bactérie appartenant au genre Streptomyces comprenant au moins une séquence nucléotidique SEQ ID NO :1 ou SEQ ID NO :8 ou toute séquence nucléotidique ayant au moins 70%, de préférence 80%, très préférentiellement 90% d'identité avec la séquence SEQ ID NO :1 ou toute séquence ayant au moins 80%, de préférence 90% d'identité avec la séquence SEQ ID NO :8.

2. Procédé selon la revendication 1 comprenant :
1) la culture d'une bactérie appartenant au genre Streptomyces comprenant au moins une séquence nucléotidique SEQ ID NO :1 ou SEQ ID NO :8 ou toute séquence nucléotidique ayant au moins 70%, de préférence 80%, très préférentiellement 90% d'identité avec la séquence SEQ ID NO :1 ou toute séquence ayant au moins 80%, de préférence 90% d'identité avec la séquence SEQ ID NO :8,
2) l'addition en continu ou en une seule dose d'eugénol comme substrat de bioconversion dans le milieu de fermentation dans une quantité comprise entre 5 à 50g/l, de préférence 10 à 35 g/l,
3) l'extraction de la vanilline du milieu de fermentation.

3. Procédé selon la revendication 1, comprenant :
1) la culture d'une bactérie appartenant au genre Streptomyces comprenant au moins une séquence nucléotidique SEQ ID NO :1 ou SEQ ID NO :8 ou toute séquence nucléotidique ayant au moins 70%, de préférence 80%, très préférentiellement 90% d'identité avec la séquence SEQ ID NO :1 ou toute séquence ayant au moins 80%, de préférence 90% d'identité avec la séquence SEQ ID NO :8,
2) l'addition en une seule dose d'eugénol comme substrat de bioconversion dans le milieu de fermentation dans une quantité comprise entre 5 à 40g/l, de préférence 10 à 35 g/l,
3) l'extraction de l'alcool coniférylique du milieu de fermentation.

4. Procédé selon la revendication 1, comprenant :
1) la culture d'une bactérie appartenant au genre Streptomyces comprenant au moins une séquence nucléotidique SEQ ID NO :1 ou SEQ ID NO :8 ou toute séquence nucléotidique ayant au moins 70%, de préférence 80%, très préférentiellement 90% d'identité avec la séquence SEQ ID NO :1 ou toute séquence ayant au moins 80%, de préférence 90% d'identité avec la séquence SEQ ID NO :8,
2) l'addition en continu d'eugénol comme substrat de bioconversion dans le milieu de fermentation dans une quantité comprise entre 5 à 30g/l, de préférence 10 à 25 g/l,
3) l'extraction de l'acide férulique du milieu de fermentation.

5. Procédé selon l'une quelconque des revendications 1 à 4, dans lequel ladite bactérie est incubée dans un milieu dont le pH est inférieur à 10.

6. Procédé selon l'une quelconque des revendications 1 à 5, dans lequel ladite bactérie est incubée à une température comprise entre 27 et 40°C, de préférence égale à 37°C.

7. Procédé selon la revendication 1, dans lequel ladite bactérie est obtenue par :
a) clonage de la séquence nucléotidique SEQ ID NO :1 ou SEQ ID NO :8 dans un vecteur d'expression, de préférence spécifique des bactéries appartenant au genre Streptomyces,
b) transformation du vecteur d'expression obtenu en a) dans une bactérie compétente, de préférence E. Coli DH5α,
c) transfert du vecteur d'expression comprenant la séquence SEQ ID NO :°1 ou SEQ ID NO : 8 dans E.Coli ET12567,
d) transformation ou conjugaison d'une bactérie appartenant au genre Streptomyces avec la bactérie obtenue en c),
e) sélection des meilleurs transformants obtenus en d) en présence du substrat eugénol.

8. Bactérie appartenant au genre Streptomyces comprenant au moins une séquence nucléotidique SEQ ID NO :1 ou SEQ ID NO : 8 ou toute séquence nucléotidique ayant au moins 70%, de préférence 80%, très préférentiellement 90% d'identité avec la séquence SEQ ID NO :1 ou toute séquence ayant au moins 80%, de préférence 90% d'identité avec la séquence SEQ ID NO :8.

9. Bactérie selon la revendication 8 étant de préférence Streptomyces griseus.

10. Séquence nucléotidique SEQ ID NO :1.

11. Séquence nucléotidique SEQ ID NO :8.

12. Vecteur d'expression comprenant au moins une séquence SEQ ID NO :1 ou SEQ ID NO :8.

13. Vecteur selon la revendication 11, **caractérisé en ce qu'**il est un vecteur bifonctionnel spécifique des bactéries appartenant au genre Streptomyces.

14. Vecteur selon la revendication 12, **caractérisé en ce qu'**il est choisi parmi un vecteur intégratif ou un vecteur réplicatif de type multicopie.

15. Vecteur selon la revendication 13, **caractérisé en ce qu'**il est choisi parmi pFLA2 et pFLA3.

16. Vecteur selon la revendication 13, **caractérisé en ce qu'**il est le vecteur pFLA4.

17. Procédé selon la revendication 1, dans lequel est produit la vanilline, l'acide férulique ou l'alcool coniférylique à hauteur de 10 à 25 g/l.

18. Procédé selon la revendication 1, dans lequel la conversion de l'acide férulique en vanilline s'effectue dans la même bactérie appartenant au genre Streptomyces ou s'effectue par voie biochimique ou enzymatique.

## Claims

1. Method for the production of natural ferulic acid, coniferyl alcohol and/or vanillin, comprising the bioconversion of eugenol by a bacterium belonging to the genus Streptomyces comprising at least one nucleotide sequence SEQ ID NO:1 or SEQ ID NO:8 or any nucleotide sequence having at least 70%, preferably 80%, very preferably 90%, identity with the sequence SEQ ID NO:1 or any nucleotide sequence having at least 80%, preferably 90% identity with the sequence SEQ ID NO:8.

2. Method according to claim 1 comprising:
1) the culture of a bacterium belonging to the genus Streptomyces comprising at least one nucleotide sequence SEQ ID NO:1 or SEQ ID NO:8 or any nucleotide sequence having at least 70%, preferably 80%, very preferably 90%, identity with the sequence SEQ ID NO:1 or any nucleotide sequence having at least 80%, preferably 90% identity with the sequence SEQ ID NO:8,
2) the addition, continuously or in a single dose, of eugenol as a bioconversion substrate to the fermentation medium in a quantity comprised between 5 and 50 g/l, preferably 10 to 35 g/l,
3) the extraction of the vanillin from the fermentation medium.

3. Method according to claim 1, comprising:
1) the culture of a bacterium belonging to the genus Streptomyces comprising at least one nucleotide sequence SEQ ID NO:1 or SEQ ID NO:8 or any nucleotide sequence having at least 70%, preferably 80%, very preferably 90%, identity with the sequence SEQ ID NO:1 or any nucleotide sequence having at least 80%, preferably 90% identity with the sequence SEQ ID NO:8,
2) the addition, in a single dose, of eugenol as a bioconversion substrate to the fermentation medium in a quantity comprised between 5 and 40 g/l, preferably 10 to 35 g/l,
3) the extraction of the coniferyl alcohol from the fermentation medium.

4. Method according to claim 1, comprising:
1) the culture of a bacterium belonging to the genus Streptomyces comprising at least one nucleotide sequence SEQ ID NO:1 or SEQ ID NO:8 or any nucleotide sequence having at least 70%, preferably 80%, very preferably 90%, identity with the sequence SEQ ID NO:1 or any nucleotide sequence having at least 80%, preferably 90% identity with the sequence SEQ ID NO:8,
2) the continuous addition of eugenol as a bioconversion substrate to the fermentation medium in a quantity comprised between 5 and 30 g/l, preferably 10 to 25 g/l,
3) the extraction of the ferulic acid from the fermentation medium.

5. Method according to any one of claims 1 to 4, wherein said bacterium is incubated in a medium wherein the pH is less than 10.

6. Method according to any one of claims 1 to 5, wherein said bacterium is incubated at a temperature comprised between 27 and 40 °C, preferably equal to 37 °C.

7. Method according to claim 1, wherein said bacterium is obtained by:
a) cloning of the nucleotide sequence SEQ ID NO:1 or SEQ ID NO:8 in an expression vector, preferably specific to the bacteria belonging to the genus Streptomyces,
b) transformation of the expression vector obtained in a) in a competent bacterium, preferably E. coli DH5α,
c) transfer of the expression vector comprising the sequence SEQ ID NO:1 or SEQ ID NO:8 in E. coli ET12567,
d) transformation or conjugation of a bacterium belonging to the genus Streptomyces with the bacterium obtained in c),
e) selection of the best transformants obtained in d) in the presence of the eugenol substrate.

8. Bacterium belonging to the genus Streptomyces comprising at least one nucleotide sequence SEQ ID NO:1 or SEQ ID NO:8 or any nucleotide sequence having at least 70%, preferably 80%, very preferably 90%, identity with the sequence SEQ ID NO:1 or any nucleotide sequence having at least 80%, preferably 90% identity with the sequence SEQ ID NO:8.

9. Bacterium according to claim 8 being preferably Streptomyces griseus.

10. Nucleotide sequence SEQ ID NO:1.

11. Nucleotide sequence SEQ ID NO:8.

12. Expression vector comprising at least one sequence SEQ ID NO:1 or SEQ ID NO:8.

13. Vector according to claim 11, wherein the vector is a bifunctional vector specific to the bacteria belonging to the genus Streptomyces.

14. Vector according to claim 12, wherein the vector is chosen from an integrative vector or a replicative vector of the multicopy type.

15. Vector according to claim 13, wherein the vector is chosen from the integrative vectors pFLA2 and pFLA3.

16. Vector according to claim 13, wherein the vector is the vector pFLA4.

17. Method according to claim 1, wherein vanillin, ferulic acid or coniferyl alcohol is produced at a level of 10 to 25 g/l.

18. Method according to claim 1, wherein the ferulic acid is converted to vanillin in the same bacterium belonging to the genus Streptomyces or by biochemical or enzymatic route.

## Patentansprüche

1. Verfahren zur Produktion von natürlicher Ferulasäure, von natürlichem Coniferylalkohol und/oder von natürlichem Vanillin, umfassend die Biokonversion von Eugenol durch ein Bakterium, das zur Gattung Streptomyces gehört und das mindestens eine Nukleotidsequenz SEQ ID NO :1 oder SEQ ID NO :8 oder eine beliebige Nukleotidsequenz mit mindestens 70%, vorzugsweise 80%, sehr stark bevorzugt 90% Identität zu der Sequenz SEQ ID NO :1 aufweist oder jegliche Sequenz, die mindestens 80%, bevorzugt 90%, Identität zu der Sequenz SEQ ID NO :8 aufweist, umfasst.

2. Verfahren nach Anspruch 1, das Folgendes umfasst:
1) Kultur eines Bakteriums, das zur Gattung Streptomyces gehört und das mindestens eine Nukleotidsequenz SEQ ID NO :1 oder SEQ ID NO :8 oder eine beliebige Nukleotidsequenz mit mindestens 70%, vorzugsweise 80%, sehr stark bevorzugt 90% Identität zu der Sequenz SEQ ID NO :1 aufweist oder jegliche Sequenz, die mindestens 80%, bevorzugt 90%, Identität zu der Sequenz SEQ ID NO :8 aufweist, umfasst,
2) kontinuierliches Zugeben oder Zugeben in einer Portion von Eugenol als Substrat für die Biokonversion in dem Fermentationsmedium in einer Menge von 5 bis 50 g/l, vorzugsweise 10 bis 35 g/l,
3) Extraktion des Vanillins aus dem Fermentationsmedium.

3. Verfahren nach Anspruch 1, das Folgendes umfasst:
1) Kultur eines Bakteriums, das zur Gattung Streptomyces gehört und das mindestens eine Nukleotidsequenz SEQ ID NO :1 oder SEQ ID NO :8 oder eine beliebige Nukleotidsequenz mit mindestens 70%, vorzugsweise 80%, sehr stark bevorzugt 90% Identität zu der Sequenz SEQ ID NO :1 aufweist oder jegliche Sequenz, die mindestens 80%, bevorzugt 90%, Identität zu der Sequenz SEQ ID NO :8 aufweist, umfasst,
2) Zugeben in einer Portion von Eugenol als Substrat für die Biokonversion in dem Fermentationsmedium in einer Menge von 5 bis 40 g/l, vorzugsweise 10 bis 35 g/l,
3) Extraktion des Coniferylalkohols aus dem Fermentationsmedium.

4. Verfahren nach Anspruch 1, das Folgendes umfasst:
1) Kultur eines Bakteriums, das zur Gattung Streptomyces gehört und das mindestens eine Nukleotidsequenz SEQ ID NO :1 oder SEQ ID NO :8 oder eine beliebige Nukleotidsequenz mit mindestens 70%, vorzugsweise 80%, sehr stark bevorzugt 90% Identität zu der Sequenz SEQ ID NO :1 aufweist oder jegliche Sequenz, die mindestens 80%, bevorzugt 90%, Identität zu der Sequenz SEQ ID NO :8 aufweist, umfasst,
2) kontinuierliches Zugeben von Eugenol als Substrat für die Biokonversion in dem Fermentationsmedium in einer Menge von 5 bis 30 g/l, vorzugsweise 10 bis 25 g/l,
3) Extraktion der Ferulasäure aus dem Fermentationsmedium.

5. Verfahren nach einem der Ansprüche 1 bis 4, wobei das Bakterium in einem Medium inkubiert wird, dessen pH-Wert unter 10 liegt.

6. Verfahren nach einem der Ansprüche 1 bis 5, wobei das Bakterium bei einer Temperatur zwischen 27 und 40°C, vorzugsweise bei einer Temperatur von 37°C, inkubiert wird.

7. Verfahren nach Anspruch 1, wobei das Bakterium durch Folgendes erhalten wird:
a) Klonieren der Nukleotidsequenz SEQ ID NO :1 oder SEQ ID NO :8 in einem Expressionsvektor, vorzugsweise einem für Bakterien, die zur Gattung Streptomyces gehören, spezifischen Expressionsvektor,
b) Transformation des in a) erhaltenen Expressionsvektors in ein kompetentes Bakterium, vorzugsweise E. Coli DH5α,
c) Transfer des Expressionsvektors, der die SEQ ID NO :1 oder SEQ ID NO :8 umfasst, in E. coli ET12567,
d) Transformation oder Konjugation eines Bakteriums, das zur Gattung Streptomyces gehört, mit dem in c) erhaltenen Bakterium,
e) Selektion der besten in d) erhaltenen Transformanten in Gegenwart des Substrats Eugenol.

8. Bakterium, das zur Gattung Streptomyces gehört und das mindestens eine Nukleotidsequenz SEQ ID NO :1 oder SEQ ID NO :8 oder eine beliebige Nukleotidsequenz mit mindestens 70%, vorzugsweise 80%, sehr stark bevorzugt 90% Identität zu der Sequenz SEQ ID NO :1 aufweist oder jegliche Sequenz, die mindestens 80%, bevorzugt 90%, Identität zu der Sequenz SEQ ID NO :8 aufweist, umfasst.

9. Bakterium nach Anspruch 8, bei dem es sich vorzugsweise um Streptomyces griseus handelt.

10. Nukleotidsequenz SEQ ID NO :1.

11. Nukleotidsequenz SEQ ID NO :8.

12. Expressionsvektor, der mindestens eine Sequenz SEQ ID NO :1 oder SEQ ID NO :8 umfasst.

13. Vektor nach Anspruch 11, **dadurch gekennzeichnet, dass** es sich um einen für Bakterien, die zur Gattung Streptomyces gehören, spezifischen Shuttle-Vektor handelt.

14. Vektor nach Anspruch 12, **dadurch gekennzeichnet, dass** er aus der Reihe integrierender Vektor oder replizierender Vektor des Multicopy-Typs ausgewählt ist.

15. Vektor nach Anspruch 13, **dadurch gekennzeichnet, dass** er aus der Reihe pFLA2 und pFLA3 ausgewählt ist.

16. Vektor nach Anspruch 13, **dadurch gekennzeichnet, dass** es sich um den Vektor pFLA4 handelt.

17. Verfahren nach Anspruch 1, bei dem das Vanillin, die Ferulasäure oder der Coniferylalkohol in einer Menge von 10 bis 25 g/l produziert wird.

18. Verfahren nach Anspruch 1, bei dem die Konversion der Ferulasäure zu Vanillin in demselben Bakterium, das zur Gattung Streptomyces gehört, erfolgt oder auf biochemischem oder enzymatischem Weg erfolgt.


### SEQUENCE LISTING

<110> V MANE FILS
<120> système de production de molécules aromatiques chez Streptomyces
<130> QT
<160> 8
<170> PatentIn version 3.3
<210> 1
   <211> 1680
   <212> DNA
   <213> artificial
<220>
   <223> VAO optimisée
<400> 1
   atgtcgaaga cccaagagtt ccggccgctg accctgccgc cgaagctgtc gctgtcggac     60
   ttcaacgagt tcatccagga catcatccgc atcgtcggct cggagaacgt cgaggtcatc    120
   tcgtcgaagg accagatcgt cgacggctgc tacatgaagc cgacccacac ccacgacccg    180
   caccacgtca tggaccagga ctacttcctg gcctcggcca tcgtcgcccc gcgcaacgtc    240
   gccgacgtgc agtcgatcgt gggcctcgcc aacaagttct cgttcccgct ctggccgatc    300
   tcgatcggcc gcaactcggg ctacggcggc gccgccccgc gcgtctcggg ctcggtcgtc    360
   ctggacatgg gcaagaacat gaaccgcgtc ctggaggtca acgtcgaggg cgcctactgc    420
   gtcgtcgagc cgggcgtcac ctaccacgac ctgcacaact acctggaggc caacaacctg    480
   cgcgacaagc tgtggctgga cgtcccggac ctgggcggcg gctcggtcct gggcaacgcc    540
   gtcgagcgcg gcgtcggcta caccccgtac ggcgaccact ggatgatgca ctcgggcatg    600
   gaggtggtgc tggccaacgg cgagctgctg cgcaccggca tgggcgccct gccggacccc    660
   aagcgccccg agaccatggg cctgaagcct gaggaccagc cgtggtcgaa gatcgcccac    720
   ctgttcccgt acggcttcgg cccgtacatc gacggcctgt tctcgcagtc gaacatgggc    780
   atcgtgacca agatcggcat ctggctgatg ccgaacccgg gcggctacca gtcgtacctg    840
   atcaccctgc cgaaggacgg cgacctgaag caggccgtcg acatcatccg cccgctgcgc    900
   ctgggcatgg ccctgcagaa cgtcccgacc atccgccaca tcctgctgga cgccgccgtc    960
   ctgggcgaca agcgctcgta ctcgtcgaag accgagccgc tgtcggacga ggagctggac   1020
   aagatcgcca agcagctgaa cctgggccgc tggaacttct acggcgccct gtacggcccg   1080
   gagccgatcc gccgcgtgct gtgggagacc atcaaggacg ccttctcggc catcccgggc   1140
   gtgaagttct acttcccgga ggacaccccg gagaactcgg tcctgcgcgt ccgcgacaag   1200
   accatgcagg gcatcccgac ctacgacgag ctgaagtgga tcgactggct gccgaatggc   1260
   gcccacctgt tcttctcgcc gatcgccaag gtctcgggcg aggacgccat gatgcagtac   1320
   gccgtcacca agaagcgctg ccaggaggcc ggcctggact tcatcggcac cttcaccgtg   1380
   ggcatgcgcg agatgcacca catcgtctgc atcgtcttca acaagaagga cctgatccag   1440
   aagcgcaagg tccagtggct gatgcgcacg ctgatcgacg actgcgccgc caacggttgg   1500
   ggcgagtacc gcacgcacct ggccttcatg gaccagatca tggagacgta caactggaac   1560
   aactcgtcgt tcctgcgctt caacgaggtc ctgaagaacg ccgtcgaccc gaacggcatc   1620
   atcgccccgg gcaagtcggg cgtctggccg tcgcagtact cgcacgtcac ctggaagctg   1680
<210> 2
   <211> 24
   <212> DNA
   <213> artificial
<220>
   <223> amorce bla1F
<400> 2
   ctcgagagac gaactccttg aacc 24
<210> 3
   <211> 22
   <212> DNA
   <213> artificial
<220>
   <223> amorce bla1R
<400> 3
   ggccttacca atgcttaatc ag 22
<210> 4
   <211> 22
   <212> DNA
   <213> artificial
<220>
   <223> amorce tsr1F
<400> 4
   gagctctgac tgagttggac ac 22
<210> 5
   <211> 20
   <212> DNA
   <213> artificial
<220>
   <223> amorce tsr 1R
<400> 5
   ctcgacttat cggttggccg 20
<210> 6
   <211> 23
   <212> DNA
   <213> artificial
<220>
   <223> amorce rep1F
<400> 6
   acatgtgtta gtgcgaagtg ggc 23
<210> 7
   <211> 12
   <212> DNA
   <213> artificial
<220>
   <223> amorce rep1R
<400> 7
   ctgcgagttc ag 12
<210> 8
   <211> 1810
   <212> DNA
   <213> artificial
<220>
   <223> VAO optimisée 2
<400> 8
   gatctagaga tatcgagaag ggagcggaca tacatgtcga agacccaaga gttccggccg     60
   ctgaccctgc cgccgaagct gtcgctgtcg gacttcaacg agttcatcca ggacatcatc    120
   cgcatcgtcg gctcggagaa cgtcgaggtc atctcgtcga aggaccagat cgtcgacggc    180
   tcgtacatga agccgaccca cacccacgac ccgcaccacg tcatggacca ggactacttc    240
   ctggcctcgg ccatcgtcgc cccgcgcaac gtcgccgacg tgcagtcgat cgtgggcctc    300
   gccaacaagt tctcgttccc gctctggccg atctcgatcg gccgcaactc gggctacggc    360
   ggcgccgccc cgcgcgtctc gggctcggtc gtcctggaca tgggcaagaa catgaaccgc    420
   gtcctggagg tcaacgtcga gggcgcctac tgcgtcgtcg agccgggcgt cacctaccac    480
   gacctgcaca actacctgga ggccaacaac ctgcgcgaca agctgtggct ggacgtcccg    540
   gacctgggcg gcggctcggt cctgggcaac gccgtcgagc gcggcgtcgg ctacaccccg    600
   tacggcgacc actggatgat gcactcgggc atggaggtgg tgctggccaa cggcgagctg    660
   ctgcgcaccg gcatgggcgc cctgccggac cccaagcgcc ccgagaccat gggcctgaag    720
   cctgaggacc agccgtggtc gaagatcgcc cacctgttcc cgtacggctt cggcccgtac    780
   atcgacggcc tgttctcgca gtcgaacatg ggcatcgtga ccaagatcgg catctggctg    840
   atgccgaacc cgggcggcta ccagtcgtac ctgatcaccc tgccgaagga cggcgacctg    900
   aagcaggccg tcgacatcat ccgcccgctg cgcctgggca tggccctgca gaacgtcccg    960
   accatccgcc acatcctgct ggacgccgcc gtcctgggcg acaagcgctc gtactcgtcg   1020
   aagaccgagc cgctgtcgga cgaggagctg gacaagatcg ccaagcagct gaacctgggc   1080
   cgctggaact tctacggcgc cctgtacggc ccggagccga tccgccgcgt gctgtgggag   1140
   accatcaagg acgccttctc ggccatcccg ggcgtgaagt tctacttccc ggaggacacc   1200
   ccggagaact cggtcctgcg cgtccgcgac aagaccatgc agggcatccc gacctacgac   1260
   gagctgaagt ggatcgactg gctgccgaat ggcgcccacc tgttcttctc gccgatcgcc   1320
   aaggtctcgg gcgaggacgc catgatgcag tacgccgtca ccaagaagcg ctgccaggag   1380
   gccggcctgg acttcatcgg caccttcacc gtgggcatgc gcgagatgca ccacatcgtc   1440
   tgcatcgtct tcaacaagaa ggacctgatc cagaagcgca aggtccagtg gctgatgcgc   1500
   acgctgatcg acgactgcgc cgccaacggt tggggcgagt accgcacgca cctggccttc   1560
   atggaccaga tcatggagac gtacaactgg aacaactcgt cgttcctgcg cttcaacgag   1620
   gtcctgaaga acgccgtcga cccgaacggc atcatcgccc cgggcaagtc gggcgtctgg   1680
   ccgtcgcagt actcgcacgt cacctggaag ctgggatccc accaccacca ccaccacgga   1740
   tcctaataat tactaattaa ttggggaccc tagaggtccc ctttttattt ttaaaagata   1800
   tcaagcttgc                                                          1810